# EUROPÄISCHE PATENTSCHRIFT

(11) **EP 3 318 297 B1**
(45) Veröffentlichungstag und Bekanntmachung des Hinweises auf die Patenterteilung: **25.09.2024**
(21) Anmeldenummer: 17200299.0
(22) Anmeldetag: 07.11.2017
(51) Int. Cl.: A61M 16/20

(54) **EXPIRATIONSVENTIL**
EXPIRATION VALVE
SOUPAPE D'EXPIRATION

(30) Priorität: 08.11.2016 DE 102016121380
(43) Veröffentlichungstag der Anmeldung: 09.05.2018
(73) Patentinhaber: Hamilton Medical AG, 7402 Bonaduz (CH)
(72) Erfinder: Breitruck, Felix, 9470 Buchs SG (CH); De-Stefani, Dino, 7000 Chur (CH)
(74) Vertreter: Schmitt-Nilson Schraud Waibel Wohlfrom Patentanwälte Partnerschaft mbB

(56) Entgegenhaltungen:
- WO-A1-00/45883
- WO-A1-2015/127994
- US-A- 4 699 137
- US-A- 5 005 568
- US-A1- 2009 293 878
- US-A1- 2013 119 289
- ANONYMOUS: "Expiratory Valve Set, autoclavable", HAMILTON MEDICAL REPROCESSING GUIDE, 1 January 2017 (2017-01-01), United States, pages 1 - 172, XP055437521, ISBN: 978-1-55581-255-3

## Beschreibung

Die vorliegende Erfindung betrifft ein Expirationsventil eines Beatmungsgeräts, aufweisend einen Ventilkörper, in dem ein Fluideinlass und ein Fluidauslass für Atemluft eines Patienten ausgebildet sind, wobei der Ventilkörper einen Fluid - strömungsweg zwischen dem Fluideinlass und dem Fluidauslass definiert, dessen Querschnitt sich zum Fluidauslass hin erweitert.

Dieses Expirationsventil wird bei einem Beatmungsgerät zur maschinellen Beatmung von Patienten eingesetzt. Die vorliegende Erfindung betrifft auch ein sol - ches Beatmungsgerät.

Bei der maschinellen Beatmung wird einem Patienten Atemluft auf maschinellem Wege, in der Regel unter einem Überdruck zugeführt. Die maschinelle Beatmung kann die eigene Atmung des Patienten dabei unterstützen oder vollständig er - setzten. Das Zuführen der Beatmungsluft erfolgt in aufeinander folgenden Beat - mungszyklen. Jeder Beatmungszyklus hat eine Inspirationsphase mit nachfolgender Expirationsphase. Dabei wird die Beatmungsluft während der Inspirationspha - se eines jeweiligen Beatmungszyklus zugeführt, in der Regel maschinell oder je - denfalls maschinell unterstützt, und mit einem Überdruck gegenüber dem in den Atemwegen des Patienten herrschenden Druck. Das Ausatmen erfolgt während einer nachfolgenden Expirationsphase des Beatmungszyklus. Hierbei erfolgt in der Regel keine Beaufschlagung der Atemwege mit Überdruck oder Unterdruck. Vielmehr soll das Ausatmen passiv erfolgen durch Entspannen der Atemwege ge - genüber dem Umgebungsdruck, wobei eigene Ausatmungsanstrengungen des Patienten soweit als möglich unterstützt werden sollen. Das Ausatmen der Atem - luft und die Abgabe der Atemluft an die Umgebung soll mit Hilfe des hier be - schriebenen Expirationsventils erfolgen. Das Expirationsventil ist im Beatmungssystem in einer Leitung für ausgeatmete Luft angeordnet, in der Regel am strom - abwärts liegenden Ende dieser Leitung. Auf seiner vom Patienten abgewandten Seite herrscht in der Regel Umgebungsdruck. Wird es während einer Expirations - phase mit Überdruck gegenüber diesem Druck von der Seite des Patienten her beaufschlagt, so soll das Expirationsventil öffnen und eine Abgabe der ausgeatmeten Luft an die Umgebung ermöglichen. Das Expirationsventil soll dabei Beginn und Ende von Expirationsphasen zuverlässig zu erkennen und entsprechend reagieren. Hierzu dient eine Ventilmembran, die je nachdem, ob auf ihrer den Atemwegen zugewandten Seite ein genügend großer Überdruck gegenüber ihrer der Umgebung zugewandten Seite herrscht, einen Fluiddurchgang zwischen den Atemwegen der Umgebung freigibt oder verschließt.

WO 00/45883 A1 zeigt ein Beatmungsventil mit einem Einlass, durch den Atemluft von einem Beatmungsgerät in das Beatmungsventil einleitbar ist, einem ersten Auslass, durch den Atemluft zu einem Patienten ausgeleitet und Ausatemluft von einem Patienten eingeleitet wird, einem zweiten Auslass, durch den die Ausatemluft des Patienten in einen Hohlraum des Beatmungsventils eingeleitet wird, und Öffnungen, die in dem Hohlraum angeordnet sind, und durch die die Ausatemluft dann aus dem Beatmungsventil ausgeleitet wird. Eine Membran trennt den zweiten Auslass von dem Hohlraum, so dass bei einer Beatmung die Atemluft von dem Beatmungsgerät nicht über die Öffnungen aus dem Beatmungsventil entweicht.

WO 2015/127994 A1 zeigt einen Durchflussmesser eines Beatmungsgeräts mit einem in einem Gehäuse zentral angeordneten Rohr. Das Rohr hat einen Einlassbereich und einen Auslassbereich. Das Gehäuse weist ferner einen Ausleitkanal auf, welcher mit dem Auslassbereich des Rohrs verbunden ist. Eine flexible Membran ist über dem Auslassbereich angeordnet und verschließt diesen.

US 5 005 568 A zeigt ein Isolationsventil, welches in Beatmungsgeräten zum Einsatz kommt. Das Isolationsventil weist ein Einlassrohr und ein Auslassrohr auf, welche durch eine Membran voneinander getrennt sind. Die Membran weist einen Port auf, der bei einer Beatmung eines Patienten Luft von dem Einlassrohr zum Auslassrohr passieren lässt. Beim Ausatmen des Patienten verschließt die Membran das Einlassrohr und die Ausatemluft des Patienten wird von der Membran zu mehreren, im Querschnitt kleineren Auslassöffnungen geleitet.

US 2009/0293878 A1 zeigt eine Beatmungsvorrichtung zum Beatmen eines Patienten durch einen Luftschlauch. Die Beatmungsvorrichtung weist ein Verbindungsrohr zum Patienten auf, durch den Atemluft dem Patienten wird und Ausatemluft vom Patienten abgeführt wird. Dies geschieht durch mehrere kleinere Öffnungen, die um das Verbindungsrohr angeordnet sind.

In der Vergangenheit trat häufig das Problem auf, dass das Expirationsventil im Betrieb unangenehm laute Geräusche verursachte. In manchen Fällen wurde sogar beobachtet, dass vom Expirationsventil oszillierende Druckschwankungen in der ausgeatmeten Luft verursacht oder verstärkt wurden, die letztendlich zu Fehlsteuerungen in der Beatmung führten. Bei diesem als Autotrigger oder Autocycle bekannten Phänomen werden vom Expirationsventil verursachte oder verstärkte Druckschwankungen vom Beatmungsgerät fälschlicherweise als Ende der Expirationsphase detektiert und das Beatmungsgerät leitet vorzeitig die nächste Inspirationsphase ein. Dies ist für den zu beatmenden Patienten sehr unangenehm und kann sogar zur Gefährdung des beatmeten Patienten führen. Das erfindungsgemäße Expirationsventil soll derartige Probleme vermeiden oder jedenfalls verringnern.

Gegenüber den bisher verwendeten Expirationsventilen erlaubt das eingangs umschriebene erfindungsgemäße Expirationsventil eine verbesserte Führung der ausgeatmeten Luft. Insbesondere reagiert das Expirationsventil feinfühliger auf den Beginn und das Ende der Expirationsphase, und die Geräuschentwicklung im Betrieb des Expirationsventils ist deutlich unterdrückt. Außerdem gelingt es, das Phänomen des Autotrigger oder Autocycle besser zu beherrschen.

Das erfindungsgemäße Expirationsventil weist alle Merkmale des Anspruchs 1 auf.

Der sich zum Fluidauslass hin erweiternde Querschnitt des Fluidströmungswegs ist besonders gut an den zum Fluidauslass hin immer größer werdenden Volumenstrom des während der Expiration durch das in dieser Phase geöffnete Expirationsventil strömenden Fluids (der ausgeatmeten Luft) angepasst. Je größer der Volumenstrom, desto größer der im Fluidströmungsweg bereitstehende Querschnitt. Insbesondere verändern sich auf jeweils gleich große Winkelbereiche bezogene Teilvolumina des Fluidströmungswegs entlang eines Umfangs des Fluideinlasses, und zwar derart, dass diese Teilvolumina in Richtung zum Fluidauslass hin immer größer werden. Das vermindert die Ausbildung von Turbulenz und Störungen in der Fluidströmung und schafft die Möglichkeit, dass das Expirationsventil sehr viel zuverlässiger, aber gleichzeitig auch empfindlicher, auf Veränderungen der Strömung ausgeatmeter Luft in dem Atemwegen regieren kann. Druck-Rückschläge, die bei herkömmlichen Expirationsventilen häufig zu den genannten Autotrigger-Fehlsteuerungen führten, treten sehr viel weniger in Erscheinung.

Der Querschnitt des Fluidströmungswegs soll als Fläche des für die Fluidströmungzur Verfügung stehenden Raums in quer, insbesondere orthogonal, zur hauptsächlichen Strömungsrichtung der Fluidströmung zwischen Fluideinlass und Fluidauslass bei geöffnetem Expirationsventil stehenden Schnittebenen verstanden werden.

Das Expirationsventil ist so ausgebildet, dass Atemluft beim Ausatmen, also während der Expirationsphase, durch den nunmehr geöffneten Fluideinlass in das Expirationsventil, insbesondere in ein vom Expirationsventil umschlossenes Ventilplenum, eindringen kann. Während der Inspirationsphase ist der Fluideinlass vorzugsweise verschlossen.

Der Fluidauslass ist so ausgebildet, dass ausgeatmete Atemluft in eine Umgebung abgebbar ist.

Erfindungsgemäß bildet der Ventilkörper ein topfförmiges Ventilplenum, welches wenigstens zum Teil an seiner Unterseite von dem Ventilkörper, insbesondere von einem Ventilboden und ggf. von einem vom Ventilboden ausgehenden und ebenfalls vom Ventilkörper gebildeten äußeren Rand, umschlossen ist und in das der Fluideinlass mündet. An seiner Oberseite kann das Ventilplenum von einer einen Deckel bildenden Ventilmembran begrenzt sein. Das Ventilplenum bildet einen Hohlraum, der über den Fluideinlass mit Fluid (ausgeatmete Atemluft) beaufschlagbar ist und aus dem über den Fluidauslass das Fluid (ausgeatmete Atemluft) ausströmen kann. Insbesondere ist in dem Ventilplenum der Fluidströmungsweg zwischen dem Fluideinlass und dem Fluidauslass gebildet. Insbesondere ist das Ventilplenum wenigstens zum Teil an seiner Unterseite von einem Ventilboden begrenzt und der Fluideinlass mündet in den Ventilboden. Der Ventilboden kann sich dabei insbesondere tellerartig zwischen dem Fluideinlass und einem äußeren Rand des Ventilkörpers (insbesondere des Ventilplenums) erstrecken, wobei der äußere Rand des Ventilbodens zugleich den äußeren Rand des Ventilkörpers bildet. Mit dem Begriff "tellerartig" soll umschrieben sein, dass der Ventilboden sich, abgesehen von einer möglichen noch näher zu erläuternden rinnenartigen Vertiefung um den Fluideinlass herum, im Wesentlichen flach oder flach konisch zwischen dem Fluideinlass und einem äußeren Rand erstreckt. Der Fluideinlass ist dann insbesondere in einem zentralen Teil des Ventilbodens ausgebildet. Der Fluideinlass kann insbesondere einen kreisförmigen Querschnitt aufweisen mit ebenfalls kreisförmiger Fluideintrittsfläche.

Erfindungsgemäß geht der Ventilboden von einem eine Fluideintrittsfläche definierenden Rand des Fluideinlasses aus. Es entsteht kein Absatz zwischen der Fluideintrittsfäche und dem Ventilboden. Auf diese Weise werden scharfkantige Begrenzungen des Fluideinlasses gegenüber dem Ventilplenum vermieden. Das fördert die Ausbildung einer möglichst ruhigen Strömung des Fluids in das Expirationsventil sowie durch das Expirationsventil während der Expirationsphase. Bei bekannten Expirationsventilen ragt demgegenüber der Fluideinlass in das Ventilplenum hinein, es steht insbesondere der Fluideinlass ein Stück weit über den Ventilboden vor. Der Ventilboden geht dann nicht von dem die Fluideintrittsfläche definierenden Rand des Fluideinlasses aus und die Fluidströmung passiert einen Absatz beim Einströmen in das Ventilplenum, was die Ausbildung von Turbulenz und Strömungsabrissen in der in das Expirationsventil einströmenden Fluidströmung fördert.

Zur Reduzierung von Geräuschbildung und Druck-Rückwirkungen kann auch betragen, wenn das Ventilplenum entlang des Fluidströmungswegs zwischen Fluideinlass und Fluidauslass möglichst strömungsgünstig ausgelegt ist. Die strömungsgünstige Form des Ventilplenums, insbesondere des Fluidströmungswegs kann insbesondere verbessert werden, wenn der Ventilboden ausgehend von einer Innenseite des Fluideinlasses am Rand der Fluideintrittsfläche auf seiner dem Ventilplenum zugewandten Seite eine abgerundete Kontur aufweist. Die vom Fluideinlass her in das Ventilplenum einströmende Fluidströmung gelangt so weitgehend unverwirbelt und ohne Strömumgsabrisse in den Fluidströmungsweg und strömt dem Fluidauslass zu. Dies vermindert Betriebsgeräusche bei geöffnetem Fluidauslass unmittelbar. Außerdem werden Druck-Rückschlage in die an dem Fluideinlass angeschlossene Atemluftleitung effektiv unterdrückt, so dass die Gefahr von Fehl-Erfassungen eines Endes der Expirationsphase durch das Beatmungsgerät geringer wird.

In der strömungsgünstigen Ausgestaltung des Ventilplenums, insbesondere derart, das entlang des im Ventilplenum ausgebildeten Fluidströmungswegs vom Fluideinlass zum Fluidauslass nur abgerundete Konturen gebildet sind, wird ein eigenständig schützenswerter Gegenstand der vorliegenden Erfindung gesehen. Die Anmelderin behält sich vor, darauf einen eigenständigen Schutz zu beanspruchen, unabhängig von der Konfiguration des Fluidströmungswegs mit sich zum Fluidauslass hin erweiterndem Querschnitt.

Erfindungsgemäß wölbt sich der Ventilboden in einem Bereich um den Fluideinlass herum in axialer Richtung aus. Mit der Formulierung "in axialer Richtung auswölbt" soll dabei ausgedrückt werden, dass der Ventilboden in wenigstens einem eine Fluideinlassachse enthaltenden Schnitt einen konvexen Verlauf hat, und zwar in einem zwischen dem Rand des Fluideinlasses und dem Außenrand des Ventilbodens liegenden Bereich. Konvex bedeutet, dass in einem solchen Schnitt eine zwei Punkte auf dem Ventilboden verbindende Linie durch das Ventilplenum verläuft. Der Ventilboden bildet demgemäß eine um den Fluideinlass umlaufende Rinne aus, deren Boden sich zwischen dem Rand des Fluideinlasses und dem Außenrand des Ventilbodens in axialer Richtung auswölbt. Der Querschnitt des Fluidströmungswegs zwischen dem Fluideinlass und dem Fluidauslass ergibt sich bei solcher Ausgestaltung jeweils als Fläche des Ventilplenums in einem von der Fluideinlassachse ausgehenden und bis zum äußeren Rand des Ventilplenums reichenden radialen Schnitt. Im Gegensatz dazu ist der Ventilboden bei derzeit Verwendung findenden Expirationsventilen flach konisch ausgebildet, er bildet also keine Auswölbung.

Erfindungsgemäß hat die axiale Auswölbung eine sich um den Umfang des Fluideinlasses herum verändernde Tiefe. Die Tiefe der Auswölbung lässt sich dabei als ein in axialer Richtung gemessenen Abstand zwischen dem Ventilboden am Rand des Fluideinlasses und dem Ventilboden an der tiefsten Stelle der Auswölbung entlang eines Schnitts vom Rand des Fluideinlasses zum äußeren Rand des Ventilbodens bestimmen. Insbesondere soll die Tiefe der Auswölbung in Umfangsrichtung zu dem Fluidauslass hin zunehmen. Damit verändern sich auf jeweils gleich große Winkelbereiche bezogene Teilvolumina der Auswölbung entlang des Umfangs des Fluideinlasses, und zwar derart, dass diese Teilvolumina in Richtung zum Fluidauslass hin immer größer werden. In bestimmten Ausführungsformen kann die Tiefe der Auswölbung in Umfangsrichtung auf einer dem Fluidauslass gegenüber liegenden Seite am kleinsten sein und in Umfangsrichtung auf der zum Fluidauslass hin gelegenen Seite am größten sein.

Wie bereits angesprochen, kann der Fluideinlass insbesondere in einem zentra - len Bereich des Ventilplenums angeordnet sein. Der Fluidauslass kann bezogen auf den Fluideinlass weiter außen angeordnet sein, insbesondere in radialer Richtung weiter außen, insbesondere an einem äußeren Rand des Ventilplenums. Der Querschnitt des Fluidströmungswegs zwischen dem Fluideinlass und dem Fluidauslass ergibt sich bei zentraler Anordnung des Fluideinlasses jeweils als Querschnittsfläche des Ventilplenums in einem jeweiligen von der Fluideinlassachse ausgehenden und bis zum äußeren Rand des Ventilplenums reichenden radialen Schnitt.

Der Ventilkörper kann insbesondere aus Kunststoffmaterial hergestellt sein. Es bietet sich beispielsweise an, den Ventilkörper im Spritzgussverfahren herzustellen. Dann ist der Ventilkörper ein Spritzgussteil. Beispielsweise kann der Ventilkörper einschließlich des Ventilbodens aus einem spritzgussfähigen Kunststoff - material hergestellt sein, etwa Polyethylen, Polypropylen oder einem anderen thermoplastischen Kunststoffmaterial.

In bestimmten Ausführungsformen kann der Ventilkörper einen Fluideinlassteil aufweisen, in dem der Fluideinlass ausgebildet ist. Insbesondere kann der Fluideinlassteil integral mit dem Ventilkörper ausgebildet sein, also ebenfalls als Spritzgussteil für den Fall, dass der Ventilkörper ein Spritzgussteil ist. Der Fluideinlassteil kann sich insbesondere von dem Ventilboden weg erstrecken, und zwar insbesondere von einer Unterseite des Ventilbodens weg. Der Fluideinlassteil kann sich beispielsweise orthogonal zu dem Ventilboden erstrecken. Die Flui - deinlassachse bezeichnet eine gedachte Linie in Längsrichtung des Fluidein - lasssteils, die bezüglich des Fluideinlassteils zentral liegt. Bei orthogonal zum Fluideinlass stehender Fluideintrittsfläche steht die Fluideinlassachse orthogonal zu der durch den Fluideinlass definierten Fluideintrittsfläche und liegt bezüglich der Fluideintrittsfläche zentral. In bestimmten Ausführungsformen kann der Fluideinlasssteil einen ersten zapfenartigen Ansatz ausbilden, der zum Anschluss ei - nes Atemluftschlauches ausgebildet sein kann. Beispielsweise kann der erste zapfenartige Ansatz zylindrisch oder konisch geformt sein und insbesondere als ein zylindrischer oder konischer Rohrstutzen ausgebildet sein, in welchem der Fluideinlass ausgebildet ist. Der Fluideinlass kann insbesondere einen kreisförmigen Querschnitt aufweisen.

In bestimmten Ausführungsformen kann der Ventilkörper wenigstens eine um den Fluideinlass herum ausgebildete Versteifungsrippe aufweisen. Der Ventilkörper kann dabei insbesondere mehrere um den Fluideinlass herum ausgebildete Ver - steifungsrippen aufweisen. Die mehreren Versteifungsrippen können insbesonde - re in bestimmten Abständen zueinander um einen Umfang des Fluideinlasses herum aufeinander folgen. Ein einzige Versteifungsrippe kann beispielsweise als ein den Fluideinlass umgebender Vollkörper ausgebildet sein. Die Versteifungsrip - pe(n) sind insbesondere auf einer Unterseite des Ventilbodens ausgebildet, also auf einer vom Fluidströmungsweg abgewandten Seite des Ventilbodens, so dass sie die Fluidströmung zwischen dem Fluideinlass und dem Fluidauslass nicht stören.

Die Versteifungsrippe bzw. die Versteifungsrippen sind geeignet, den Ventilboden zu versteifen, insbesondere in dem sich auswölbenden Bereich, der den Fluidein - lass umgibt. Vorsehen von Versteifungsrippe(n) führt zu einer Erhöhung des Flä - chenträgheitsmoments des Ventilkörpers, insbesondere des Ventilbodens, über den Querschnitt des Fluidströmungswegs hinweg. Der Ventilkörper wird in diesem Bereich verwindungsstreifer, so dass die in das Ventilplenum einströmende und entlang des Fluidströmungswegs strömende Fluidströmung weniger Schwingun - gen des Ventilkörpers, insbesondere des Ventilbodens, anregt. Dadurch wird vor allem Geräuschentwicklung unterdrückt, es entstehen aber auch viel weniger Druck-Rückwirkungen, die sich in der am Fluideinlass angeschlossenen Atemwegsleitung bemerkbar machen.

Es ist günstig, wenn die wenigstens eine Versteifungsrippe integral mit dem Ven - tilkörper ausgebildet ist. Integral ist so verstehen, dass die wenigstens eine Versteifungsrippe und der Ventilkörper als ein Bauteil hergestellt sind, beispielsweise dass die wenigstens eine Versteifungsrippe im selben Spritzgussprozess hergestellt ist wie der Ventilkörper, insbesondere als Einkomponentenspritzgussteil.

Günstigerweise kiann die wenigstens eine Versteifungsrippe bezogen auf einen Rand des Fluideinlasses nach außen weg verlaufen. Nach außen weg soll dabei die Richtung der wenigstens einen Versteifungsrippe bezeichnen, die so angeord - net ist, dass sie den Bereich des Ventilbodens zwischen dem Rand des Fluidein - lasses und dem äußeren Rand des Ventilbodens möglichst effektiv versteift. Ins - besondere die wenigstens eine Versteifungsrippe bezüglich einer Mitte eines Querschnitts des Fluideinlasses in radialer Richtung verlaufen. Es ist aber auch denkbar, dass die wenigstens eine Versteifungsrippe nicht genau in radialer Richtung verläuft, sondern zusätzlich in Umfangsrichtung, beispielsweise spiralförmig auf den äußeren Rand des Ventilbodens zu. Insbesondere kann die wenigstens eine Versteifungsrippe außerhalb des Rands des Fluideinlasses angeordnet sein.

Die wenigstens eine Versteifungsrippe kann wenigstens eine erste Versteifungs - rippe und wenigstens eine zweite Versteifungsrippe umfassen. Die wenigstens eine erste Versteifungsrippen ist eine innere Versteifungsrippe, die radial innerhalb der zweiten Versteifungsrippen angeordnet ist. Die wenigstens eine zweite Versteifungsrippe ist demgemäß eine äußere Versteifungsrippe. Die wenigstens eine erste Versteifungsrippe verläuft insbesondere vom Rand des Fluideinlasses entlang des Ventilbodens nach außen. Die wenigstens eine zweite Versteifungs - rippe verläuft insbesondere von einem äußeren Rand des Ventilbodens nach in - nen. Die wenigstens eine erste Versteifungsrippe verläuft insbesondere zwischen dem Rand des Fluideinlasses und dem sich auswölbenden Bereich des Ventilbo - dens. Die wenigstens eine zweite Versteifungsrippe verläuft insbesondere zwi - schen dem sich auswölbenden Bereich des Ventilbodens und einem äußeren Rand des Ventilbodens. Insbesondere endet die wenigstens eine zweite Verstei - fungsrippe in einem am äußeren Rand des Ventilbodens ausgebildeten flanschar - tigen Ansatz. Der Ansatz kann beispielsweise mindestens einen um den Rand des Ventilbodens umlaufenden Absatz aufweisen, vorzugsweise mindestens zwei im Abstand zueinander angeordnete Absätze, wobei die wenigstens eine zweite Versteifungsrippe in den Absatz, bzw. einen inneren der Absätze, übergeht.

Weiterhin kann insbesondere der Ventilkörper einen Fluidauslassteil bilden, in dem der Fluidauslass ausgebildet ist. Der Fluidauslassteil kann insbesondere einen zweiten zapfenartigen Ansatz ausbilden. Der Fluidauslassteil kann insbe - sondere integral mit dem Ventilkörper ausgebildet sein, beispielsweise ebenfalls als Spritzgussteil. Der zweite zapfenartige Ansatz kann insbesondere zylindrisch oder konisch geformt sein. Falls gewünscht, kann der zweite zapfenartige Ansatz beispielsweise als ein zylindrischer oder konischer Rohrstutzen ausgebildet sein, in welchem der Fluidauslass ausgebildet ist. Oft wird der zweite Ansatz aber unmittelbar in die Umgebung münden. Der Fluidauslass kann insbesondere einen kreisförmigen Querschnitt aufweisen.

Der sich auswölbende Bereich des Ventilbodens, welcher den Fluidströmungsweg zwischen Fluideinlass und Fluidauslass definiert, kann in den Fluidauslassteil übergehen. Der sich auswölbende Bereich des Ventilbodens kann insbesondere in eine Seitenwand des Fluidauslassteils übergehen. Insbesondere hat am Übergang zum Fluidauslassteil die durch den sich auswölbenden Ventilboden definier - te Rinne eine maximale Tiefe.

Wie der Fluideinlassteil kann sich der Fluidauslassteil insbesondere vom Ventilbo - den weg erstrecken, insbesondere von einer Unterseite des Ventilbodens weg. Der Fluideinlassteil kann sich orthogonal zu dem Ventilboden erstrecken.

Weiterhin ist es günstig, wenn der Ventilkörper ferner eine Auflage aufweist, an deren Oberseite eine Ventilmembran mit einer an ihrem äußeren Rand ausgebildeten Anlagefläche auflegbar ist, wobei die die Auflage auf ihrer Oberseite un - eben ausgebildet ist.

Wie bereits beschrieben, weist das Expirationsventil ferner eine dem Fluideinlass zugeordnete Ventilmembran auf, die mindestens zwischen einer den Fluideinlass verschließenden Stellung und einer den Fluideinlass öffnenden Stellung verlager - bar ist. Die Ventilmembran kann ohne eigenen Antrieb ausgebildet sein, so dass sie lediglich durch Druckunterschiede zwischen ihrer dem Fluideinlass zugewand - ten Seite und ihrer vom Fluideinlass abgewandten Seite zwischen Verschlussstel - lung und Freigabestellung verlagerbar ist. Zusätzlich oder alternativ kann aber auch ein Aktor vorgesehen sein, der die Ventilmembran zwischen Verschlussstellung und Freigabestellung hin und her verlagert. Dieser Aktor wird vorzugsweise vom Beatmungsgerät angesteuert. Diese Ventilmembran liegt mit der an ihrem äußeren Rand ausgebildeten Anlagefläche auf der der Oberseite der Auflage auf. Das Expirationsventil weist insbesondere ferner ein Spannelement auf, beispiels - weise einen Spannring, welcher mit dem Ventilkörper derart zusammenwirkt, dass das Spannelement in Spanneingriff oder Klemmeingriff mit der Ventilmembran ge - langt. Durch diesen Spanneingriff, der insbesondere am äußeren Umfang der Ventilmembran durch Klemmen mit dem Spannelement erfolgt, wird die Ventilmembran am Ventilkörper fixiert. Die Ventilmembran kann dabei durch das Spannelement so am Ventilkörper fixiert sein, dass sie bei Überdruck auf ihrer dem Fluideinlass zugeordneten Seite gegenüber ihrer dem Ventilplenum zugeord - neten Seite in eine den Fluideinlass gegenüber dem Ventilplenum freigebende Stellung bringbar ist. Solange das nicht der Fall ist, insbesondere bei Druckgleich - heit oder Unterdruck auf ihrer dem Fluideinlass zugeordneten Seite gegenüber ihrer dem Ventilplenum zugeordneten Seite verbleibt die Ventilmembran in einer den Fluideinlass gegenüber dem Ventilplenum verschließenden Stellung.

Die vorliegende Erfindung betrifft darüber hinaus ein Beatmungsgerät, welches ein an eine Expirationsluftleitung anschließbares Expirationsventil der vorange - hend beschriebenen Art aufweist.

Die Erfindung wird nachfolgend anhand eines Ausführungsbeispiels unter Bezug - nahme auf die Figuren näher erläutert. Es zeigt:
Fig. 1: Eine Beatmungsmaschine zur maschinellen Beatmung von Patienten, mit Steuereinheit, Monitor, sowie Schläuchen für zugeführte Beatmungsluft und für ausgeatmete Luft;
Fig. 2: Ein Expirationsventil gemäß einer Ausführungsform in Schnittansicht;
Fig. 3: Den Ventilkörper des Expirationsventils aus Fig. 2 in einer perspektivischen Ansicht von unten; und
Fig. 4: Den Ventilkörper des Expirationsventils aus Fig. 2 in einer perspektivischen Ansicht von oben.

In allen Figuren werden für jeweils gleiche oder funktionsgleiche Bauteile diesel - ben Bezugszeichen verwendet. Eine detaillierte Beschreibung solcher Bauteile erfolgt jeweils nur für die erste Figur, in der das jeweilige Bezugszeichen erscheint. Es versteht sich, dass dieselbe Beschreibung auch für alle anderen Figu - ren gilt, in denen das mit demselben Bezugszeichen versehene Bauteil zu sehen ist, es sei dann, dass ausdrücklich anderes vermerkt ist.

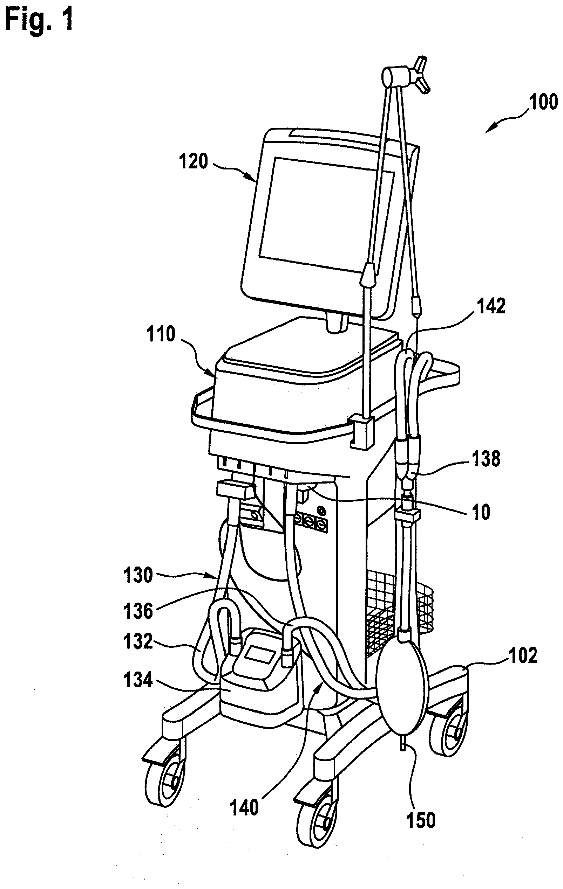

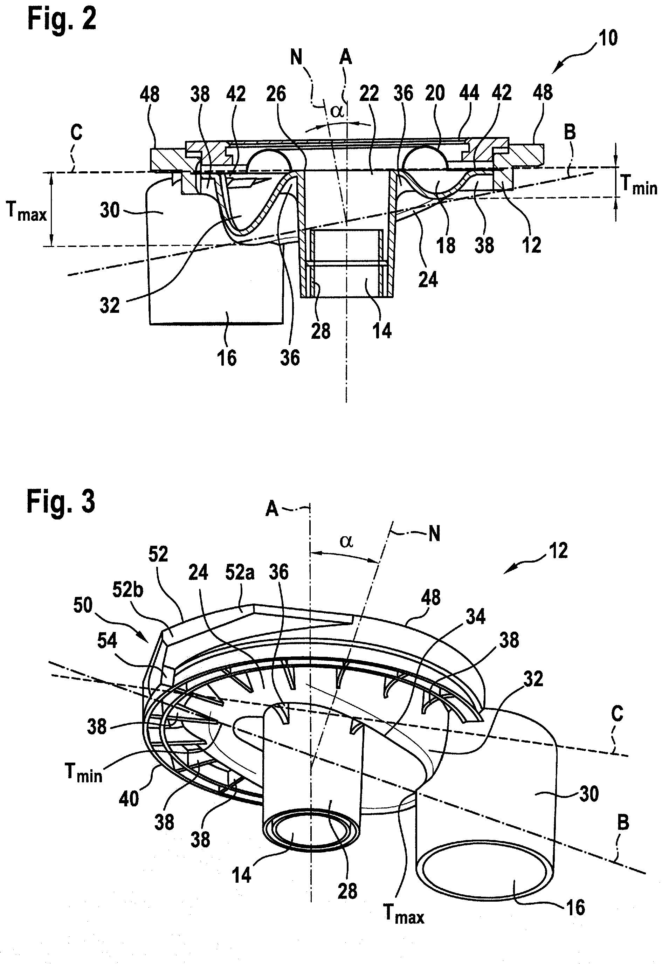

Fig. 1 zeigt eine Beatmungsmaschine 100 zur maschinellen Beatmung von Patienten. Die Beatmungsmaschine 100 umfasst ein auf Rollen fahrbares Gestell 102 mit Steuereinheit 110 und Monitor 120 sowie eine Leitung 130 für von der Maschi - ne zugeführte Beatmungsluft und eine Leitung 140 für ausgeatmete Luft. Die Lei - tung 130 für zugeführte Beatmungsluft umfasst einen von der Steuereinheit 110 ausgehenden ersten Beatmungsluftschlauch 132, der zu einer Befeuchtungsein - heit 134 führt, wo die Beatmungsluft durch ein Wasserreservoir geleitet wird. Von der Befeuchtungseinheit 134 führt ein zweiter Beatmungsluftschlauch 136 zu ei - nem T-Stück 138. Von dem T-Stück gelangt die von der Beatmungsmaschine 100 während einer Inspirationsphase des Beatmungszyklus bereitgestellte Beatmungsluft über eine Leitung 150 zum Patienten. Über die Leitung 150 gelangt auch die vom Patienten während einer Expirationsphase des Beatmungszyklus ausgeatmete Luft zurück zur Beatmungsmaschine 100. Hierzu zweigt von dem T-Stück 138 ein weiterer Atemluftschlauch 142 ab, der zu der Leitung 140 für aus - geatmete Luft gehört. Der Atemluftschlauch 142 führt zu einem allgemein mit 10 bezeichneten Expirationsventil, welches nachfolgend unter Bezugnahme auf die Figuren 2 bis 4 noch näher erläutert werden wird.

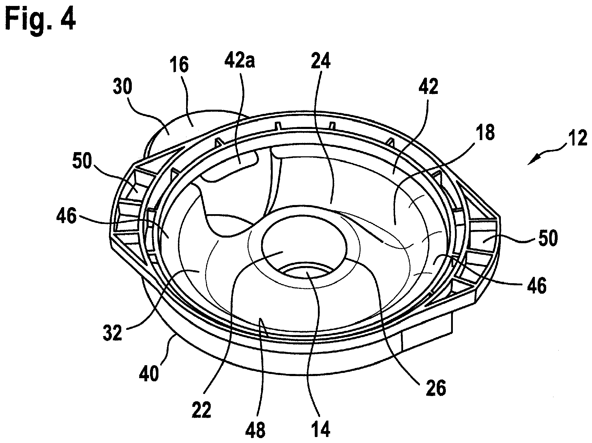

Fig. 2 zeigt in Schnittansicht das in Fig. 1 eingesetzte Expirationsventil 10 gemäß einer Ausführungsform. Das Expirationsventil 10 umfasst einen Ventilkörper 12, in dem ein Fluideinlass 14 und ein Fluidauslass 16 für Atemluft, die von einem Pati - enten ausgeatmet wird, ausgebildet sind. Die Fig. 3 und 4 zeigen perspektivische Ansichten dieses Ventilkörpers 12, und zwar zeigt Fig. 3 eine Ansicht von unten in etwa aus Richtung des mit in Fig. 4 mit 12 bezeichneten Pfeils, und zeigt Fig. 4 eine Ansicht von oben in etwa aus Richtung des in Fig. 3 mit 12 bezeichneten Pfeils. In Fig. 2 ist das Expirationsventil 10 vor dem Einbau in eine Beatmungsmaschine 100 gezeigt, so dass der Atemluftschlauch 142 noch nicht angeschlos - sen ist. Im betriebsgemäßen Zustand, d.h. nach Einbau des Expirationsventils 10 in eine Beatmungsmaschine 100 gemäß Fig. 1, ist der Atemluftschlauch 142 an dem Fluideinlass 14 angeschlossen. Somit gelangt vom Patienten ausgeatmete Luft über den Fluideinlass 14 zum Expirationsventil 10. Nach Durchgang durch das Expirationsventil 10 gelangt die ausgeatmete Luft über den Fluidauslass 16 in eine Umgebung des Beatmungsgeräts 100.

Das Expirationsventil 10 umfasst ein sogenanntes Ventilplenum 18. Das Ventilple - num 18 bildet einen Hohlraum, der über den Fluideinlass 14 mit Fluid (ausgeatmete Atemluft) beaufschlagbar ist und aus dem über den Fluidauslass 16 Fluid (ausgeatmete Atemluft) ausströmen kann. In dem Ventilplenum 18 ist somit durch den Ventilkörper 12 und eine Ventilmembran 20 ein Fluidströmungsweg zwischen dem Fluideinlass 14 und dem Fluidauslass 16 gebildet. Der Fluideinlass 14 ist so ausgebildet, dass Atemluft beim Ausatmen, d.h. während einer Expirationsphase des Beatmungszyklus, in das Ventilplenum 18 gelangen kann. Während einer In - spirationsphase des Beatmungszyklus, d.h. wenn dem Patienten über die Beatmungsleitung 130 maschinell Atemluft zugeführt wird, soll der Fluideinlass 14 verschlossen sein. Hierfür dient die dem Fluideinlass 14 zugeordnete Ventilmembran 20. Die Ventilmembran 20 ist mindestens zwischen einer den Fluideinlass 14 verschließenden Stellung und einer den Fluideinlass 14 öffnenden Stellung verlager - bar. Die Ventilmembran 20 ist insbesondere so ausgebildet, dass ein zentraler Bereich der Ventilmembran 20 eine vom Fluideinlass 14 gebildete Fluid eintrittsöffnung 22 verschließend am Fluid einlass 14 anliegt, solange der Druck auf der dem Fluideinlass 14 zugeordneten Seite der Ventilmembran 20 nicht um einen vorbestimmten Druck größer ist als auf der auf der vom Fluideinlass 14 abgewandten Seite der Ventilmembran 20 herrschende Druck (das ist normalerweise der Umgebungsdruck). Die Ventilmembran 20 befindet sich dann in einer den Fluideinlass 14 bzw. die Leitung 140 gegenüber dem Ventilplenum 18 verschlie - ßenden Stellung. Diese Stellung nimmt die Ventilmembran 20 während der Inspi - rationsphasen ein. Bei genügend großem Überdruck auf ihrer dem Fluideinlass 14 bzw. der Leitung 140 für ausgeatmete Atemluft zugeordneten Seite gegenüber ihrer vom Fluideinlass 14 bzw. der Leitung 140 für ausgeatmete Atemluft abgewandten Seite bewegt sich die Ventilmembran 20 in eine den Fluideinlass 14 ge - genüber dem Ventilplenum 18 freigebende Stellung, so dass Atemluft aus der Lei - tung 140 für ausgeatmete Atemluft in das Ventilplenum 18 gelangen kann. Dies geschieht üblicherweise während der Expirationsphasen. Die Ventilmembran 20 ist aus einem Kunststoffmaterial hergestellt, insbesondere aus einem Kunststoff - material mit elastischen Eigenschaften, beispielsweise einem Silikonkunststoff. Dabei kann die Ventilmembran 20 so eingebaut sein, dass sie ohne Beaufschla - gung durch einen Überdruck auf ihrer der Leitung 140 für ausgeatmete Atemluft zugewandten Seite unter Vorspannung an einem die Fluideintrittsöffnung 22 um - gebenden Rand 26 des Fluideinlasses 14 anliegt. Bei Beaufschlagung mit einem genügend großen Überdruck auf ihrer der Leitung 140 für ausgeatmete Atemluft zugewandten Seite löst sich die Ventilmembran 20 von dem die Fluideintrittsöffnung 22 umgebenden Rand 26 des Fluideinlasses 14, so dass ausgeatmete Atemluft in das Ventilplenum 18 gelangen kann. Zusätzlich oder alternativ kann aber auch ein Aktor vorgesehen sein, der die Ventilmembran 20 zwischen Verschlussstellung und Freigabestellung hin und her verlagert. Dieser Aktor wird vorzugsweise vom Beatmungsgerät angesteuert und greift beispielsweise in dem zentralen Bereich der Ventilmembran 20 auf der von der Fluid eintrittsöffnung 22 abgewandten Seite der Ventilmembran 20 an. Die jeweilige Stellung der Ventil - membran 20 bestimmt dabei das Volumen des Ventilplenums 18 und den zwischen Ventilmembran 20 und Ventilkörper 12 gebildeten Fluidströmungsweg.

Der Ventilkörper 12 weist einen Ventilboden 24 auf, der sich auf einer Unterseite des Ventilplenums 18 von dem Fluideinlass 14 ausgehend nach außen bis zu einem äußeren Rand des Ventilplenums bzw. Ventilbodens erstreckt. Der Ventilboden 24 definiert zusammen mit der Ventilmembran 20 den im Ventilplenum 18 gebildeten Fluidströmungsweg zwischen dem Fluideinlass 14 und dem Fluidauslass 16. Der Ventilkörper 12 bildet erfindungsgemäß ein topfförmiges Ventilplenum 18, welches wenigstens zum Teil an seiner Unterseite von dem Ventilboden 24 begrenzt ist und in das der Fluideinlass 14 mündet. Der Ventilboden 24 kann sich dabei insbesondere tellerartig zwischen dem Fluideinlass 14 und einem äußeren Rand des Ventilkörpers 12 (insbesondere des Ventilplenums 18) erstrecken, wobei der äußere Rand des Ventilbodens 24 zugleich den äußeren Rand des Ventilkörpers 12 bildet. Der Fluideinlass 14 ist dann insbesondere in einem zentralen Teil des Ventilbodens 24 ausgebildet. Der Fluideinlass 14 kann insbesondere einen kreisförmigen Querschnitt aufweisen mit ebenfalls kreisförmiger Fluideintrittsfläche 22. Ein die Fluid-eintrittsfläche 22 definierender Rand 26 des Fluideinlasses 14 bildet damit einen inneren Rand des Ventilbodens 24, der sich ausgehend von dem Rand 26 an der Unterseite des Ventilplenums 18 bis zu einem äu-ßeren Rand des Ventilkörpers 12 erstreckt. Der äußere Rand des Ventilkörpers 12 befindet sich in radialer Richtung am weitesten vom Fluideinlass 14 weg. Damit steht der Rand 26 nicht - oder jedenfalls nicht abrupt oder scharfkantig - vom Ventilboden 24 hervor, sondern vielmehr beginnt der Ventilboden 24 unmittelbar am Rand 26 der Fluideintrittsöffnung 22 und erstreckt sich in einer sanft geschwungenen Kontur von diesem Rand 26 aus nach außen. Durch diese Formgebung werden scharfe Kanten vermieden, die bei Umströmung mit Luft zu Strömungsabrissen und damit verbundener Geräuschentwicklung führen könnten.

Der Ventilboden 24 hat eine tellerartige Gestalt. Damit soll zum Ausdruck gebracht werden dass der Ventilboden 24 sich im Wesentlichen flach oder allenfalls flach konisch ausgebildet zwischen seinem inneren Rand 26 und dem äußeren Rand erstreckt. Etwaige Vertiefungen im Ventilboden 24, beispielsweise eine nachfolgend noch näher zu beschreibende Rinne, sind so ausgebildet, dass sich eine insgesamt abgerundete und strömungsangepasste Kontur ergibt mit sanft ineinander übergehenden unterschiedlichen Krümmungen.

Während der Fluideinlass 14 in einem zentralen Bereich des Ventilplenums 18 angeordnet ist, liegt der Fluidauslass 16 bezogen auf den Fluideinlass 14 radial weiter außen. Insbesondere ist der Fluidauslass 16 an einem äußeren Rand des Ventilplenums 18 angeordnet. An der Stelle des Ventilauslasses 16 ist im Ventil - körper 12, insbesondere in einer äußeren Wand des Ventilbodens 24 eine Aus - trittsöffnung ausgebildet durch die Atemluft aus dem Ventilplenum 18 zum Fluid - auslass 16 strömen kann. Auch die Austrittsöffnung ist mit sanft geschwungenen und damit strömungsgünstig ausgebildeten Kanten versehen, so dass ein Ent - langströmen von Atemluft an scharfen Kanten beim Austritt aus dem Ventilplenum 18 vermieden wird.

Der Ventilkörper 12 ist aus Kunststoffmaterial hergestellt, beispielsweise aus Polyethylen, Polypropylen oder einem anderen thermoplastischen Kunststoffmateri - al. Der Ventilkörper 12 ist ein Spritzgussteil, d.h. er ist in einem Spritzgussverfahren hergestellt. Insbesondere kann der Ventilkörper 12 so ausgebildet sein, dass er in einem einzigen Spritzgussprozess herstellbar ist, insbesondere als ein Einkomponenten-Spritzgussteil.

Der Ventilkörper 12 weist einen Fluideinlassteil 28 auf, in dem der Fluideinlass 14 ausgebildet ist. Der Fluideinlassteil 28 ist insbesondere integral mit dem Ventilkörper 12 ausgebildet. Der Fluideinlassteil 28 erstreckt sich von dem Ventilboden 24 weg, und zwar in Richtung von einer Unterseite des Ventilbodens 24 weg im We - sentlichen entlang einer Achsrichtung A des Fluideinlasses 14. Zudem erstreckt sich der Fluideinlassteil 28 quer zu dem Ventilboden 24, d.h. er steht von einer Unterseite des Ventilbodens 24 in Achsrichtung A des Fluideinlasses 14 vor. Die Achsrichtung A bezeichnet eine Fluideinlassachse, d.h. eine gedachte Linie in Längsrichtung des Fluideinlasssteils 28, die bezüglich des Fluideinlasses 14 zen - tral liegt. Bei orthogonal zum Fluideinlass 14 stehender Fluideintrittsfläche 22 steht die Fluideinlassachse A orthogonal zu der durch den Fluideinlass 14 defi - nierten Fluideintrittsfläche 22 und liegt bezüglich der Fluideintrittsfläche 22 zen - tral.

Der Fluideinlasssteil 28 bildet einen ersten zapfenartigen Ansatz aus, der insbe - sondere zum Anschluss eines Atemluftschlauches, beispielsweise des in Fig. 1 zu sehenden Atemluftschlauches 142, ausgebildet ist. In der in Fig. 2 gezeigten Vari - ante ist der erste zapfenartige Ansatz insbesondere zylindrisch geformt. In anderen Varianten könnte der zapfenartige Ansatz auch sich zu seinem freien Ende hin leicht verjüngend ausgebildet sein, beispielsweise leicht konisch zulaufend. Der erste zapfenartige Ansatz kann beispielsweise als ein Rohrstutzen ausgebil - det sein, in welchem der Fluideinlass 14 ausgebildet ist.

Der Ventilkörper 12 bildet zudem einen Fluidauslassteil 30, in dem der Fluidauslass 16 ausgebildet ist. Der Fluidauslassteil 30 kann insbesondere einen zweiten zapfenartigen Ansatz ausbilden, der zylindrisch oder sich verjüngend (insbeson - dere konisch) geformt sein kann. Der zweite zapfenartige Ansatz kann beispielsweise als ein zylindrischer Rohrstutzen ausgebildet sein, in welchem der Fluidauslass 16 ausgebildet ist. Auch der Fluidauslassteil 30 kann insbesondere inte - gral mit dem Ventilkörper 12 ausgebildet sein. Der Fluidauslass 16 kann insbesondere einen kreisförmigen Querschnitt aufweisen. Er braucht nicht unbedingt an eine weitere Leitung angeschlossen zu sein, sondern kann direkt in die Umge - bung münden, so dass ausgeatmete Luft über den Lufteinlass 16 in die Umge - bung abgegeben wird. An seiner radial inneren Seite ist der Fluidauslassteil 16 über die bereits angesprochene Austrittsöffnung mit dem Ventilplenum 18 verbun - den, so dass in das Expirationsventil 10 eingeströmtes Fluid über die Austrittsöffnung in den Fluidauslassteil 30 gelangt.

Ähnlich wie der Fluideinlassteil 28 kann sich der Fluidauslassteil 30 insbesondere vom Ventilboden 24 weg erstrecken, insbesondere von einer Unterseite des Ven - tilbodens 24 weg. Der Fluidauslassteil 30 kann sich orthogonal zu dem Ventilbo - den 24 erstrecken und/oder in einer Richtung parallel zu dem Fluideinlassteil 28 erstrecken, wie das in den Fig. 2 bis 4 gut zu sehen ist.

Der Fluidströmungsweg in dem Ventilplenum 18 besitzt einen quer zur Fluidströmung zwischen Fluideinlass 14 und Fluidauslass 16 gemessenen Querschnitt, der sich von dem Fluideinlass 14 zum Fluidauslass 16 hin erweitert. Dies wird erreicht durch eine entsprechende Ausbildung und Anordnung des Ventilkörpers 12, insbesondere des Ventilbodens 24, und der Ventilmembran 20. Der Ventilboden 24 bildet vor allem eine bodenseitige Begrenzung des Fluidströmungswegs. Die Ventilmembran 20 bildet vor allem eine Begrenzung des Fluidströmungswegs auf dessen Oberseite. Während durchaus eine Ventilmembran 20 verwendet werden kann, die herkömmlichen Ventilmembranen ähnlich ist, ist der Ventilboden 24 so ausgestaltet, dass sich der Querschnitt des Fluidströmungswegs zum Ventilauslass 16 hin vergrößert. Insbesondere ist der Ventilboden 24 so ausgebildet dass sich sein Abstand zur Ventilmembran 20 vom Fluideinlass 14 her zum Fluideinlass 16 hin vergrößert. Dies kann in einer oder mehreren Stufen geschehen, insbesondere jedoch kontiniuierlich von dem Fluideinlass 14 zu dem Fluidauslass 16 hin. Bei der in Fig. 2 bis 4 zu sehenden Geometrie des Expirationsventils 10 mit radial innen und zentral angeordnetem Fluideinlass 14 und radial außen angeord - netem Fluidauslass 16 bietet es sich beispielsweise an, dass der Fluidströmungsweg so ausgestaltet ist, dass sich auf jeweils gleich große Winkelbereiche um den Fluideinlass 14 herum bezogene Teilvolumina des Fluidströmungswegs entlang des Umfangs des Fluideinlasses 14 verändern, und zwar derart, dass diese Teilvolumina in Richtung zum Fluidauslass 16 hin immer größer werden. Ein solcher Effekt lässt sich beispielsweise erreichen, wenn eine Ebene, entlang der sich der Ventilboden 24 erstreckt, in Bezug auf eine Ebene, entlang der sich die Ventil - membran 20 erstreckt, geneigt ist, und zwar derart, dass diese beiden Ebenen zum Fluidauslass 16 hin auseinander laufen. Während sich beispielsweise die Ventilmembran 20 in aller Regel in einer Ebene orthogonal zu der Fluideinlassachse A erstrecken wird (in Fig. 2 und 3 ist diese Ebene sehr schematisch durch eine mit C bezeichnete gestrichelte Linie angedeutet), kann in bestimmten Ausführungsformen der Ventilboden 24 sich entlang einer Ebene erstrecken, die gegenüber der Fluideinlassachse A in einem von 90 Grad verschiedenen Winkel geneigt ist. Die Ebene, entlang derer sich der Ventilboden 24 erstreckt, soll dabei die Ebene bezeichnen, die der mittleren Erstreckung des Ventilbodens 24 folgt, wobei von etwaigen einzelnen (etwa dellenförmigen) Erhebungen bzw. Vertiefun - gen des Ventilbodens 24 abzusehen ist. Besitzt allerdings der Ventilboden 24 eine durchgehend ausgebildete Vertiefung, die den Querschnitt des Fluidströ - mungswegs zwischen dem Fluideinlass 14 und dem Fluidauslass 16 bestimmt, wie dies nachfolgend noch beschrieben wird, so wird die Ebene, entlang der sich der Ventilboden 24 erstreckt, im Allgemeinen durch den Verlauf dieser Vertiefung (d.h. den Verlauf der tiefsten Stellen in den einzelnen entlang des Fluidströ - mungswegs aufeinander folgenden Querschnitten) bestimmt werden. In Fig. 2 und 3 ist die Lage dieser Ebene sehr schematisch durch die Linie B angedeutet. Man sieht, dass diese Ebene nicht parallel zu der Ebene ist, entlang der die Ven - tilmembran 20 verläuft (diese Ebene verläuft orthogonal zu der Fluideinlassachse A), sondern einen von 90 Grad verschiedenen Winkel zu der Fluideinlassachse A bildet. Das impliziert, dass der Normalenvektor N dieser Ebene nicht parallel zu der Fluideinlassachse A ist, sondern einen Winkel α zu dieser bildet. Im Normalfall wird der Normalenvektor N dabei gegenüber der Fluideinlassachse A zu dem dem Fluidauslass 16 hin geneigt sein, wie das auch in Fig. 2 und 3 angedeutet ist.

Die Vergrößerung des Querschnitts des Fluidströmungswegs zum Fluidauslass 16 hin trägt dem Umstand Rechnung, dass dass der Volumenstrom an zum Auslass strömender Atemluft immer mehr zunimmt je näher der Fluidströmungsweg dem Fluidauslass liegt. Das für den Volumentstrom zur Verfügung stehende Volu - men im Ventilplenum 18 ist auf diese Weise an den Volumenstrom angepasst. Man vermeidet durch diese Anpassung des für den Fluidstrom zur Verfügung ste - henden Volumens Staueffekte mit daraus resultierender Verwirbelung der aus - zuatmenden Atemluft.

In Fig. 2 bis 4 ist zudem gut zu sehen, dass der Ventilboden 24 sich in einem Bereich um den Fluideinlass 14 herum in axialer Richtung auswölbt. Mit "in axialer Richtung auswölben" soll hierbei gemeint sein, dass der Ventilboden 24 in we - nigstens einem die Fluideinlassachse A enthaltenden Schnitt einen konvexen Ver - lauf hat, und zwar in einem zwischen dem Rand des Fluideinlasses 14 und dem äußeren Rand des Ventilbodens 24 liegenden Bereich. In den gezeigten Ausfüh - rungsformen kann dies ein in radialer Richtung verlaufender Schnitt sein. In Fig. 2 ist dieser konvexe Verlauf für den Schnitt durch die tiefste Stelle der so gebilde - ten Auswölbung 32 beim Fluidauslass 16 auf der linken Seite in Fig. 2 und für den Schnitt durch die Stelle mit geringster Tiefe der Auswölbung 32 auf der rechten Seite in Fig. 2 angedeutet. Konvex bedeutet, dass in einem solchen Schnitt eine zwei Punkte auf dem Ventilboden 24 verbindende Linie durch das Ventilplenum 18 verläuft. Der Ventilboden 24 bildet demgemäß im Bereich der so entstehenden Auswölbung 32 eine um den Fluideinlass 14 umlaufende Rinne aus, deren Boden sich zwischen dem Rand des Fluideinlasses 14 und dem äußeren Rand des Ven - tilbodens 24 in axialer Richtung auswölbt. Die Rinne definiert so den um den Flui - deinlass 14 herum führenden Fluidströmungsweg. Der entlang des Fluidströmungswegs jeweils tiefste Punkt dieser Rinne (anders ausgedrückt: die Verbin - dung der in jeweiligen Schnitten zwischen Fluideinlass 14 und äußerem Rand des Ventilbodens 24 tiefsten Punkte) bildet so eine um den Fluideinlass 14 herum füh - rende Linie, die in Fig. 3 mit 34 bezeichnet ist. Bei der in den Figuren gezeigten radialsymmetrischen Geometrie des Ventilbodens 24 mit Fluideinlass 14 in der Mitte ist diese Linie 34 wegen der noch näher zu erläuternden unterschiedlichen Tiefe der Rinne im Wesentlichen ellipsenförmig (die Projektion dieser Linie 34 auf eine zur Fluideinlassachse A orthogonale Ebene, wie die Ebene C, ist kreisför - mig). Wie in Fig. 2 bis 4 gut zu sehen ist, bildet der sich auswölbende Bereich des Ventilbodens 24 (oder die so gebildete Rinne) zwei im Wesentlichen um den halb - en Umfang des Fluideinlasses 14 herum führende Fluidströmungswege aus, die schließlich an einer Seite (in Fig. 2 links vom Fluideinlass 14) in den Fluidauslass 16 münden. Dabei geht der sich auswölbende Bereich des Ventilbodens 24 mit seiner äußeren Seitenwand in den Fluidauslassteil 30 über. Wie bereits ange - sprechen, haben alle Kanten entlang dieses Fluidströmungswegs eine abgerun - dete Kontur, die Strömungsabrissen und/oder Verwirbelungen der Fluidströmung möglichst entgegenwirkt.

In den Figuren ist gut zu sehen, dass die axiale Auswölbung 32 des Ventilbodens 24 eine sich um den Umfang des Fluideinlasses 14 herum verändernde Tiefe hat. Die Tiefe ist dabei zu messen als ein in Richtung der Fluideinlassachse A gemes - sener Abstand zwischen dem Ventilboden 24 am Rand 26 des Fluideinlasses 14 und dem Ventilboden 24 an der tiefsten Stelle der Auswölbung 32 in einem den Fluideinlass 14 mit dem äußeren Rand des Ventilbodens 24 verbindenden Schnitt (bei radialsymmetrischer Geometrie des Ventilbodens 24, wie in Figuren gezeigt, ist das ein radialer Schnitt durch den Ventilboden 24). In Fig. 2 und 3 ist die jeweils größte Tiefe Tmax an der Stelle des Fluidauslasses 16 und die jeweils kleinste Tiefe Tmin an der dem Fluidauslass 16 gegenüber liegenden Seite ange - deutet.

Auf diese Weise verändern sich auf jeweils gleich große Winkelbereiche bezoge - ne Teilvolumina des Fluidströmungswegs entlang des Umfangs des Fluideinlasses 14, und zwar derart, dass diese Teilvolumina in Richtung zum Fluidauslass 16 hin immer größer werden. Es hat sich gezeigt, dass diese Maßnahme ganz erheblich zur Reduzierung von strömungsbedingten Geräuschen beim Abgeben von Atemluft während Expirationsphasen beiträgt, weil das für die Fluidströmung zur Verfügung stehende Volumen dem Volumenstrom des strömenden Fluids an - gepasst ist.

Zudem ist in den Figuren gut zu sehen, dass der Ventilboden 24 ausgehend von einer Innenseite des Fluideinlasses 14 am Rand der Fluideintrittsfläche 22 auf seiner dem Ventilplenum 18 zugewandten Seite eine abgerundete Kontur auf - weist. Dies ist insbesondere in Fig. 4 gut zu erkennen. Der Fluidstrom strömt nach Passieren der Fluideintrittsfläche 22 entlang der abgerundeten inneren Ränder der durch die Auswölbung 32 gebildeten Rinne zu deren tiefster Stelle, um dann entlang des durch die Rinne gebildeten Fluidströmungswegs - etwa der Linie 34 folgend - um den Fluideinlass 14 herum dem Fluidauslass 16 entgegen zu strö - men. Hierbei passiert die Fluidströmung keinerlei scharfe Kanten, die zu Strömungsabrissen oder Verwirbelungen mit damit verursachter Geräuschentwicklung führen würden.

Der Ventilkörper 12 weist außerdem um den Fluideinlass 14 herum ausgebildete Versteifungsrippen 36, 38 auf. In Fig. 2 und 3 sind beispielhaft einige dieser Ver - steifungsrippen mit den Bezugszeichen 36 und 38 bezeichnet. Die Versteifungs - rippen 36, 38 versteifen den Ventilboden 24, insbesondere den durch den Ventil - boden 24 gebildeten Mantel im sich auswölbenden Bereich 32. Verwindungen des Ventilkörpers 12 werden durch diese zusätzliche Versteifung effektiv unter - drückt. Damit tragen die Versteifungsrippen 36, 38 ebenfalls dazu bei, Geräusch - entwicklung zu verringern, weil sie Schwingungen des Ventilbodens 24 unter dem Einfluss von Fluidströmung im Ventilplenum 18 unterdrücken. Damit lassen sich aber auch ungewollte störende Schwingungen der Ventilmembran 20 effektiv un - terdrücken. Dadurch sinkt die Gefahr für Oszillationen der Luftsäule im Leitungssystem, welche zu fälschlich ausgelösten Steuervorgängen (insbesondere soge - nannte Autotrigger) führen können. Die Versteifungsrippen 36, 38 sind integral mit dem Ventilkörper 12 ausgebildet. Integral ist dabei so verstehen, dass die Versteifungsrippen 26 28 und der Ventilkörper 12 als ein einziges Bauteil hergestellt sind. In dem gezeigten Ausführungsbeispiel sind die Versteifungsrippen 36, 38 im selben Spritzgussprozess hergestellt wie der Ventilkörper 12.

Die Versteifungsrippen 36, 38 sind außerhalb des Fluideinlasses 14 angeordnet. Wie in den Figuren zu sehen ist, verlaufen sie bezogen auf den Rand 26 des Fluideinlasses 14 nach außen zum Rand des Ventilkörpers 12 hin. In dem gezeigten Ausführungsbeispiel verlaufen die Versteifungsrippen bezüglich der Fluidein - lassachse A in radialer Richtung. Sie könnten aber auch eher spiralförmig von in - nen nach außen führen. Die Versteifungsrippen verbinden dabei den Rand 26 des Fluideinlasses 14 mit dem äußeren Rand des Ventilkörpers 12. Die Versteifungsrippen 36, 38 sind auf einer Unterseite des Ventilbodens 24 ausgebildet. Somit stören sie den auf der Innenseite des Ventilkörpers 12, d.h. an der Obersei - te des Ventilbodens 24 ausgebildeten Fluidströmungsweg nicht. Wie in den Figu - ren zu sehen ist, umfassen die Versteifungsrippen erste Versteifungsrippen 36 und zweite Versteifungsrippen 38. Die ersten Versteifungsrippen 36 sind innere Versteifungsrippen, die zwischen dem Rand 26 des Fluideinlasses 14 und dem sich auswölbenden Bereich 32 des Ventilbodens 24 verlaufen. In dem gezeigten Ausführungsbeispiel verbinden die ersten Versteifungsrippen 36 den Rand 26 des Fluideinlasses 14 mit dem sich auswölbenden Bereich 32 des Ventilbodens 24. Die zweiten Versteifungsrippen 38 sind äußere Versteifungsrippen, die zwi - schen dem sich auswölbenden Bereich des Ventilbodens 32 und dem äußeren Rand des Ventilbodens 24 verlaufen. In dem gezeigten Ausführungsbeispiel verbinden die zweiten Versteifungsrippen 38 den sich auswölbenden Bereich 32 des Ventilbodens 24 mit dem äußeren Rand des Ventilbodens 24. Die zweiten Ver - steifungsrippen 38 enden in einem am äußeren Rand des Ventilbodens 24 aus - gebildeten flanschartigen Ansatz 40. Der Ansatz 40 kann beispielsweise mindes - tens einen um den äußeren Rand des Ventilbodens 24 umlaufenden Absatz auf - weisen, insbesondere mindestens zwei im Abstand zueinander angeordnete Ab - sätze, wobei die zweiten Versteifungsrippen 38 in den Absatz, bzw. einen inneren der Absätze, übergehen. Der Absatz bzw. die Absätze kann beispielsweise einen ringartigen Flansch bilden, der um den äußeren Rand des Ventilbodens 24 um - läuft und diesem Stabilität verleiht.

Wie in Fig. 2 gezeigt ist, weist das Expirationsventil 10 ferner die dem Fluidein - lass 14 zugeordnete Ventilmembran 20 auf. Die Ventilmembran 20 liegt im in das Expirationsventil 10 eingebauten Zustand mit einer an ihrem äußeren Rand aus - gebildeten Anlagefläche an der Oberseite einer Auflage 42 an, die an der Ober - seite des Ventilkörpers 12 ausgebildet ist. Die Auflage 42 bildet einen um den äu - βeren Rand des Ventilkörpers 12 umlaufenden Vorsprung, der von dem äußeren Rand des Ventilkörpers 12 nach innen, zur Seite des Ventilplenums 18, vorspringt. Die Auflage 42 ist ebenfalls integral mit dem Ventilkörper 12 ausgebildet, beispielsweise als Spritzgussteil im selben Spritzgussprozess ausgebildet wie der Ventilkörper 12. Beim Zusammenbau des Expirationsventils 10 wird die Ventilmembran 20 mit ihrer äußeren Anlagefläche in Anlage gebracht mit der Oberseite der Auflage 42 des Ventilkörpers 12, so dass die Ventilmembran 20 mit einer an ihrem zentralen Teil ausgebildeten Anlagefläche auf dem Rand 26 des Fluidein - lasses 14 aufliegt und mit der an ihrem äußeren Teil ausgebildeten Anlagefläche an der Auflage 42 aufliegt. Die Ventilmembran 20 bildet so eine Oberseite des im Ventilplenum 18 gebildeten Fluidströmungswegs. Die Ventilmembran 20 wird dann mittels eines in Fig. 2 gezeigten Spannrings 44 gegenüber dem Ventilkörper 12 eingespannt. Hierzu wird der Spannring 44 auf die Ventilmembran aufgesetzt und dann bajonettartig gegenüber dem Ventilkörper 12 verdreht. Durch die so entstehenden Spannkräfte verformt sich die Ventilmembran 20 an ihrem Umfang in gewissem Umfang.

Die Auflage 42 ist deswegen auf ihrer Oberseite uneben ausgebildet. Die so ge - bildeten in Fig 4 mit dem Bezugszeichen 46 angedeuteten Unebenheiten der Oberfläche der Auflage 42 kompensieren die beim Verspannen des Spannrings 44 gegenüber dem Ventilkörper 12 entstehende Verformung der Ventilmembran 20, so dass die Bildung von Falten oder Verwerfungen am Umfang der Ventil - membran 20 im eingespannten Zustand unterdrückt werden kann. Insbesondere sind an der Oberseite der Auflage 42 Unebenheiten 46 in Gestalt von entlang des Umfangs im Abstand voneinander angeordneten Auswölbungen oder Rippen aus - gebildet. Diese Auswölbungen oder Rippen können einen radialen Verlauf haben. Noch besser ist es, wenn die Auswölbungen oder Rippen gewindeartig ausgebil - det sind, also eine gewisse Steigung gegenüber der Ebene der aufliegenden Ventilmembran 20 aufweisen und/oder nicht nur in radialer Richtung verlaufen, sondern auch in Umfangsrichtung, beispielsweise einen spiralartigen Verlauf um den Rand des Ventilkörpers 12 herum besitzen. Um die erzeugten Spannkräfte zu begrenzen, kann es zudem günstig sein, die Steigung der durch die Auswöl - bungen oder Rippen gebildeten Gewindegänge gering zu halten und/oder den ansteigenden Bereich zu begrenzen, etwa durch einen Anschlag am Ende einer Auswölbung oder Rippe und/oder durch Übergang in einen parallel zur Ebene der Ventilmembran 20 verlaufenden Bereich am Ende der Auswölbung oder Rippe. Die Unebenheiten, insbesondere in Gestalt von Auswölbungen und/oder Rippen, können einteilig mit der Auflage 42 ausgebildet sein.

Die Auflage 42 kann um den Umfang des äußeren Randes des Ventilkörpers um - laufend ausgebildet sein (wie etwa in dem mit 42 bezeichneten Bereich in Fig. 4) oder mit Unterbrechungen ausgebildet sein, so dass sich einzelnen Auflageberei - che ergeben, die entlang des Umfangs des äußeren Randes des Ventilkörpers 12 an aufeinander folgen. In dem Ausführungsbeispiel gem. Fig. 4 ist ein solcher ein - zelner Auflagebereich 42a auf der Seite des Fluideinlasses 16 oberhalb der Aus - trittsöffnung zwischen dem Ventilplenum 18 und dem Fluidauslass 16 ausgebil - det.

Die Oberseite 46 der Auflage 42 ist uneben ausgebildet, so dass die Oberseite 46 der Auflage 42 in einer gedachten gewölbten Fläche liegt, es also es keine ge - dachte Ebene gibt, die die Oberseite 46 der Auflage 42 vollständig enthält. Die gedachte gewölbte Fläche überdeckt eine Oberseite des Ventilkörpers 12. Sie liegt insbesondere orthogonal zu der Fluideinlassachse A, die zugleich eine Längsachse des Ventilkörpers 12 bildet. Wegen der unebenen Form der Obersei - te 46 der Auflage 42 würde eine ebene Fläche, die man auf die Oberseite 46 der Auflage aufzulegen versucht, nicht die gesamte Oberseite 46 der Auflage 42 be - rühren. Vielmehr würden nur einzelne Stellen der ebenen Fläche Kontakt mit der Oberseite 46 der Auflage 42 haben. Daher liegt, jedenfalls im nicht in ein Beat - mungsgerät 100 eingebauten Zustand des Expirationsventils 10 die ebene Ventil - membran 20 nicht entlang ihres gesamten Umfangs an der Oberseite 46 der Auflage 42 auf. Es gibt vielmehr einzelne Berührungsstellen zwischen Ventilmem - bran 20 und Auflage entlang des Umfangs der Ventilmembran 20. Die Unebenheit der Oberseite der Auflage 42 ist nun so ausgebildet, dass erst dann, wenn das Expirationsventil 10 in das Beatmungsgerät 100 eingebaut ist und der Ventilkörper 12 unter Spannung steht, so dass er sich deformiert, die Oberseite 46 der Auflage 42 in einer ebenen Fläche zu liegen kommt und die Ventilmembran 20 nun mit ihrer Anlagefläche flächig auf der Oberseite 46 der Auflage 42 aufliegt.

Der Ventilkörper 12 weist wenigstens eine Montagelasche 50 auf, die zum Eingriff mit einem bajonettartigen Expirationsventilanschluss eines Beatmungsgeräts (100) ausgebildet ist. Das Beatmungsgerät weist dann ein Gegeneingriffselement auf, mit dem die Montagelasche 50 beim Einbau des Expirationsventils 10 in Eingriff gelangt, um das Expirationsventil 10 am Beatmungsgerät 100 zu halten. Ein solcher Bajonettverschluss hat die Eigenschaft, dass beim Verriegeln des Expira - tionsventils 10 am Beatmungsgerät 100 Spannkräfte entstehen, die den Ventilkör - per 12 etwas deformieren.

Der Ventilkörper 12 weist wenigstens zwei Montagelaschen 50 auf, die einander zugeordnet sind. Die Montagelaschen 50 sind am radial äußeren Rand des Ven - tilkörpers 12 um einen Umfang des Ventilkörpers 12 herum angeordnet und ste - hen in radialer Richtung vom jeweiligen radial äußeren Rand des Ventilkörpers 12 hervor, um jeweilige Eingriffsflächen 52 mit einem entsprechenden Gegenein - griffselement am Beatmungsgerät 100 zu bilden. Bringt man die Montagelaschen 50 in Eingriff mit ihren jeweiligen am Beatmungsgerät 100 vorgesehenen Gegen - eingriffselementen, wird der Ventilkörper 12 auf den jeweils zugeordneten Seiten, an denen sich die Montagelaschen 50 befinden, zum Beatmungsgerät 100 hin geführt. Im Zuge dieser Zwangsbewegung entsteht eine Spannung im Ventilkörper 12, und die obere Kante 48 des Ventilkörpers 12 biegt sich auf den Seiten, an denen die Montagelaschen 50 angeordnet sind, zum Beatmungsgerät 100 hin. Die unebene Ausgestaltung der Oberseite 46 der am Ventilkörper 12 vorgesehe - nen Auflage 42 für die Ventilmembran 20 soll diese Verbiegung des Ventilkörpers 12 ausgleichen. Nach Ineingriffbringen der Montagelaschen 50 mit dem jeweili - gen Gegeneingriffselement am Beatmungsgerät 100 soll die Oberseite 46 der Auflage 42 flach sein, also in einer Ebene liegen. Das beiden Montagelaschen 50 liegen einander in einem Winkel von 180° zueinander gegenüber, d.h. auf gegen - überliegenden Seiten des Ventilkörpers 12 über das Ventilplenum 18 hinweg.

Die Oberseite 46 der Auflage 42 liegt in einer sich bezogen auf das Ventilplenum 18 nach außen gewölbten gedachten Fläche, die die Oberseite des Ventilkörpers 12 überdeckt. Insbesondere liegt die nach außen gewölbte gedachte Fläche be - zogen das Ventilplenum 18 weiter außen als eine gedachte Ebene durch wenigs - tens zwei verschiedene Punkte auf der Oberseite 46 der Auflage 42 an Seiten, an denen Montagelaschen 50 vorhanden sind (also auf der linken und rechten Seite in Fig. 4). Die Oberseite 46 der Auflage 42 liegt dabei in einer konvex gewölbten gedachten Fläche, so dass eine zwei in der gedachten Fläche liegende Punkte verbindende Linie durch das Ventilplenum 18 verläuft. Dabei wölbt sich die Ober - seite 46 der Auflage 42 ausgehend von den beiden Montagelaschen 50 nach au - ßen. An den Stellen, an denen sich die Montagelaschen 50 befinden, wölbt sich die Oberseite 46 der Auflage 42 gar nicht nach außen, wohingegen sie sich in der Mitte zwischen den beiden Montagelaschen 50 (in Fig. 4 entlang einer Linie von oben nach unten orthogonal zu einer Verbindung zwischen den beiden Montage - laschen 50) am meisten nach außen auswölbt. Dies trägt der Tatsache Rechnung, dass die Deformierung des Ventilkörpers 12 beim Befestigen des Expirati - onsventils 10 am Beatmungsgerät 100 dort am stärksten ist, wo sich die Montagelaschen 50 befinden. An dieser Stelle wird die obere Kante 48 des Ventilkör - pers 12 am stärksten nach oben gebogen.

In dem gezeigten Ausführungsbeispiel ist die gewölbte gedachte Fläche eine Mantelfläche eines Zylinders, wobei die Achse des Zylinders orthogonal zu einer Verbindungslinie zwischen den beiden zugeordneten Montagelaschen 50 ist.

Wegen der beim Befestigen des Expirationsventils 10 an Stellen, an den Monta - gelaschen 50 sind, auf den den Ventilkörper 12 ausgeübten Spannung biegt sich auch die obere Kante 48 des Ventilkörpers 12 in derselben Weise an diesen Stel - len zu einer Gegenseite am Beatmungsgerät 100 hin. Zur Kompensation dieses Effektes ist es günstig, auch den Ventilkörper 12, genauer gesagt, die obere Kante 48 des Ventilkörpers 12, in entsprechender Weise auszubilden wie die Auflage 42 für die Ventilmembran 20. Die obere Kante 48 des Ventilkörpers soll 12 nach Befestigen des Expirationsventils 10 am Beatmungsgerät 100 an einer Gegenflä - che des Beatmungsgeräts 100 anliegen und zwar in einer möglichst flächigen Weise, um eine möglichst dichte Anlage der Ventilmembran 20 zu gewährleisten. Deshalb weist der Ventilkörper 12 eine obere Kante 48 auf, welche in einer sich bezogen auf das Ventilplenum 18 nach außen gewölbten gedachten Fläche liegt. Die bereits dargelegten Überlegungen für die Oberseite 46 der Auflage 42 gelten für die Ausgestaltung der oberen Kante 48 des Ventilkörpers 12 in entsprechen - der Weise. Insbesondere liegt die obere Kante 48 des Ventilkörpers 12 in einer sich bezogen auf das Ventilplenum 18 konvex gewölbten gedachten Fläche und wölbt sich ausgehend von der wenigstens einen Montagelasche 50 aus nach au - ßen. Dabei ist die Auswölbung der oberen Kante 48 des Ventilkörpers 12 in einem zwischen den beiden Montagelaschen 50 liegenden Bereich größer als in einem einer der beiden Montagelaschen 50 benachbarten Bereich.

Die unebene Form der Oberseite 46 der Auflage 42 und die unebene Form der oberen Kante 48 des Ventilkörpers 12 sind aufeinander abgestimmt. Das ist sinn - voll, denn es soll für beide erreicht werden, dass nach Befestigen des Expirati - onsventils 10 am Beatmungsgerät 100 sowohl die Oberseite 46 der Auflage 42 als auch die obere Kante 48 des Ventilkörpers 12 in einer ebenen Fläche liegt. Insbesondere wölben sich die Oberseite 46 der Auflage 42 und die obere Kante 48 des Ventilkörpers 12 zueinander ausgerichtet nach außen, wobei die Orte mi - nimalerAuswölbung und maximaler Auswölbung aufeinander abgestimmt sind, insbesondere in Bezug auf eine das Ventilplenum 18 überdeckende Ebene an derselben Stelle liegen. Es ist sogar so, dass die Oberseite 46 der Auflage 42 und die obere Kante 48 des Ventilkörpers 12 sich in etwa gleichermaßen auswölben und insbesondere in in zueinander parallelen gedachten Flächen liegen.

Jeder der Montagelaschen 50 weist eine Eingriffsfläche 52 zum Eingriff mit einem am Beatmungsgerät 100 vorgesehenen Gegeneingriffselement auf, wobei die Eingriffsfläche 52 mit einer Einlaufschräge 52a versehen ist, die zu der Auswöl - bung der Oberseite der Anlage 42 abgestimmt ist. Wie bei einem Bajonetteingriff üblich, wird die Eingriffsfläche 52 in der Regel im Wesentlichen parallel zu einer oberen Kante der Montagelasche ausgebildet sein. In ihrem vorderen Bereich ist die Einlaufschräge 52a vorgesehen. Im Bereich der Einlaufschräge 52a verläuft die Eingriffsfläche 52 dann in einem Winkel, insbesondere einem spitzen Winkel, zu der oberen Kante der Montagelasche 50. Der Winkel ist so gewählt, dass eine Dicke zwischen Eingriffsfläche 52 und oberer Kante der Montagelasche 50 mit zunehmendem Abstand vom Beginn der Eingriffsfläche 52a zunimmt. Damit lässt sich eine gewisse Spannung beim Ineingriffbringen der Montagelasche 50 mit dem Gegeneingriffselement am Beatmungsgerät 100 erzeugen, was zu einem gewissen Kraftschluss und damit einem festen und sicheren Sitz des Expirations - ventils 10 am Beatmungsgerät 100 verhilft. Die Einlaufschräge 52a ist so ausge - bildet, dass beim Einspannen der Montagelasche 50 am Beatmungsgerät 100 eine Vorspannung erzeugt wird, die auf die obere Kante 48 des Ventilkörpers auf der Seite der Montagelasche 50 einwirkt. Damit einher geht eine gewisse Deformation des Ventilkörpers 12 auf der der Montagelasche 50 zugewandten Seite gegenüber Seiten oder Stellen weiter abseits der Montagelasche 50. Insbesondere wird die obere Kante 48 des Ventilkörpers 12 durch Bewegung des Gegenein - griffselements entlang der Einlaufschräge 52a näher zum Beatmungsgerät 100 hin geführt. Andererseits liegen weiter von der Montagelasche 50 entfernte Stel - len bereits an einer Kontaktfläche des Beatmungsgeräts 100 an und können sich nicht weiter annähern. Im Ergebnis entsteht eine Verbiegung der oberen Kante 48 des Ventilkörpers 12, wobei die Verbiegung zu der Montagelasche 50 hin immer stärker wird. Dadurch entsteht eine Vorspannung im Ventilkörper 12, die mit zunehmender Bewegung des Gegeneingriffselements entlang der Einlaufschräge 52a zunimmt. Die Einlaufschräge 52a ist so gewählt, dass beim Einspannen eine Vorspannung erzeugt wird, die gerade groß genug ist, um die obere Kante 48 des Ventilkörpers 12 im eingespannten Zustand eben zu machen, d.h. dass die obere Kante 48 des Ventilkörpers 12 im eingespannten Zustand in einer das Ventilple - num 18 überdeckenden Ebene liegt. Dann liegt die obere Kante 48 des Ventilkör - pers 12 flächig an einer Gegenfläche des Beatmungsgeräts 100 an. Gleicherma - ßen verbiegt sich auch die integral mit dem Ventilkörper 12 ausgebildete Auflage 42 für die Ventilmembran 20. Damit die Oberseite 46 dieser Auflage 42 im am Beatmungsgerät 100 befestigten Zustand des Expirationsventils 10 ebenfalls eben ist, ist es günstig, wenn die Oberseite 46 der Auflage 42 in demselben Maße un - eben ist wie die obere Kante 48 des Ventilkörpers 12. Die Einlaufschräge 52a der Montagelasche 50 ist demgemäß auf die Auswölbung der Auflage 42 abgestimmt.

Um das Ende der Einlaufschräge 52a möglichst gut zu definieren und damit die Vorspannung für den Ventilkörper 12 möglichst genau einstellen zu können, ist vorgesehen sein, dass die Eingriffsfläche 52 einen auf die Einlaufschräge 52a fol - genden nicht abgeschrägten Bereich 52b aufweist. Im nicht abgeschrägten Be - reich 52b ist die Eingriffsfläche 52 parallel zu der oberen Kante der Montagela - sche 50. Eine einmal erreichte Vorspannung und Verbiegung des Ventilkörpers 12 änderst sich damit nicht mehr, wenn das Gegeneingriffselement den nicht abge - schrägten Bereich 52b erreicht. Damit kann die Vorspannung und Verbiegung gut reproduzierbar eingestellt werden, weil es nicht genau darauf ankommt, bis zu welcher Stelle des nicht abgeschrägten Bereichs 52b die Montagelasche 50 ge - langt. Zudem ist noch die Eingriffsfläche 52 an ihrem Ende mit einem Anschlag 54 versehen, der ein Ende der Ineingriffbewegung zwischen Montagelaschen 50 und Beatmungsgerät 100 festlegt.

Das beschriebene Expirationsventil 10 kann als ein Einwegbauteil ausgebildet sein, d.h. so ausgebildet sein, dass es nur einmalig einsetzbar ist und nach Gebrauch entsorgt werden muss. Solche Expirationsventile sind billig herzustellen und erfüllen doch die geforderten Hygienestandards. Alternativ kann das Expirati - onsventil 10 auch so ausgebildet sein, dass ein mehrmalige Verwendung möglich ist. Hierfür wäre die Gestaltung des Expirationsventils 10 so auszuführen, dass es nach Gebrauch desinfizierbar ist, beispielsweise durch Autoklavieren. Das Expirationsventil ist dann autoklavierfest auszulegen.

## Patentansprüche

1. Expirationsventil (10) eines Beatmungsgeräts (100), aufweisend
einen Ventilkörper (12), in dem ein Fluideinlass (14) und ein Fluidauslass (16) für Atemluft eines Patienten ausgebildet sind, wobei der Ventilkörper (12) einen Fluidströmungsweg zwischen dem Fluideinlass (14) und dem Fluidauslass (16) definiert, dessen Querschnitt sich zum Fluidauslass (16) hin erweitert;
wobei der Ventilkörper (12) ein topfförmiges Ventilplenum (18) bildet, welches wenigstens zum Teil von einem Ventilboden (24) umschlossen ist und in das der Fluideinlass (14) mündet; und
wobei der Ventilboden (24) von einem eine Fluideintrittsfläche (22) definierenden Rand (26) des Fluideinlasses (14) ausgeht;
**dadurch gekennzeichnet, dass**
der Ventilboden (24) sich in einem Bereich um den Fluideinlass (14) herum in axialer Richtung mit einer Auswölbung (32) auswölbt. die eine sich um den Umfang des Fluideinlasses (14) herum verändernde Tiefe hat.

2. Expirationsventil (10) nach Anspruch 1, wobei der Ventilboden (24) sich tellerartig zwischen dem Fluideinlass (14) und einem Außenrand des Ventilkörpers (12) erstreckt; wobei sich der Ventilboden flach oder flach konisch zwischen dem Fluideinlass und dem Außenrand erstreckt.

3. Expirationsventil(10) nach Anspruch 1 oder 2, wobei der Ventilboden (24) ausgehend von einer Innenseite des Fluideinlasses (14) am Rand (26) der Fluideintrittsfläche (22) auf seiner dem Ventilplenum (18) zugewandten Seite eine abgerundete Kontur aufweist.

4. Expirationsventil (10) nach einem der Ansprüche 1 bis 3, wobei die Tiefe der Auswölbung (32) in Umfangsrichtung zu dem Fluidauslass (16) hin zunimmt.

5. Expirationsventil (10) nach einem der Ansprüche 1 bis 4, wobei die Tiefe der Auswölbung (32) in Umfangsrichtung auf einer dem Fluidauslass (16) gegenüber liegenden Seite kleiner ist als in allen anderen Bereichen der Auswölbung.

6. Expirationsventil (10) nach einem der Ansprüche 1 bis 5, wobei die Tiefe der Auswölbung (32) in Umfangsrichtung auf einer zum Fluidauslass (16) hin gelegenen Seite größer ist als in allen anderen Bereichen der Auswölbung.

7. Expirationsventil (10) nach einem der Ansprüche 1 bis 6, wobei der Fluideinlass (14) in einem zentralen Bereich des Ventilplenums (18) angeordnet ist.

8. Expirationsventil (10) nach Anspruch 7, wobei der der Fluidauslass (16) bezogen auf den Fluideinlass (14) weiter außen angeordnet ist.

9. Expirationsventil (10) nach einem der Ansprüche 1 bis 8, wobei der Ventilkörper (12) aus Kunststoffmaterial hergestellt ist.

10. Expirationsventil (10) nach einem der Ansprüche 1 bis 9, wobei der Ventilkörper (12) ein Spritzgussteil ist.

11. Expirationsventil (10) nach einem der Ansprüche 1 bis 10, wobei der Ventilkörper (12) einen Fluideinlassteil (28) aufweist, in dem der Fluideinlass (14) ausgebildet ist.

12. Expirationsventil (10) nach Anspruch 11, wobei der Fluideinlasssteil (28) einen ersten zapfenartigen Ansatz ausbildet; wobei der zapfenartige Ansatz entweder zylindrisch geformt ist, oder sich zu einem freien Ende hin verjüngt.

13. Expirationsventil (10) nach einem der Ansprüche 1 bis 12, wobei der Ventilkörper (12) wenigstens eine um den Fluideinlass (14) herum ausgebildete Versteifungsrippe (36, 38) aufweist, insbesondere mehrere um den Fluideinlass (14) herum ausgebildete Versteifungsrippen (36, 38).

14. Expirationsventil (10) nach Anspruch 13, wobei die wenigstens eine Versteifungsrippe (36, 38) integral mit dem Ventilkörper (12) ausgebildet ist.

15. Expirationsventil (10) nach Anspruch 14, wobei die wenigstens eine Versteifungsrippe (36, 38) bezogen auf den Rand (26) des Fluideinlasses (14) nach au-ßen weg verläuft.

16. Expirationsventil (10) nach einem der Ansprüche 13 bis 15, wobei die wenigstens eine Versteifungsrippe wenigstens eine erste Versteifungsrippe (36) umfasst, die von dem Rand (26) des Fluideinlasses (14) entlang des Ventilbodens (24) verläuft.

17. Expirationsventil (10) nach einem der Ansprüche 13 bis 16, wobei die wenigstens eine Versteifungsrippe wenigstens eine zweite Versteifungsrippe (38) umfasst, die von dem äußeren Rand des Ventilbodens (24) entlang des Ventilbodens (24) nach innen verläuft.

18. Expirationsventil (10) nach einem der Ansprüche 1 bis 17, wobei der Ventilkörper (12) einen Fluidauslassteil (30) bildet, in dem der Fluidauslass (16) ausgebildet ist.

19. Expirationsventil (10) nach Anspruch 18, wobei der sich auswölbende Bereich (32) des Ventilbodens (24) in den Fluidauslassteil (16) übergeht.

20. Expirationsventil nach einem der vorangehenden Ansprüche, ferner aufweisend eine dem Fluideinlass zugeordnete Ventilmembran, die mindestens zwischen einer den Fluideinlass verschließenden Stellung und einer den Fluideinlass öffnenden Stellung verlagerbar ist.

21. Expirationsventil (10) nach einem der Ansprüche 1 bis 20, wobei der Ventilkörper (12) ferner eine Auflage (42) aufweist, an deren Oberseite die Ventilmembran (20) mit einer an ihrem äußeren Rand ausgebildeten Anlagefläche auflegbar ist, wobei die die Auflage (42) auf ihrer Oberseite uneben ausgebildet ist.

22. Beatmungsgerät (100), aufweisend ein an eine Expirationsluftleitung (140) anschließbares Expirationsventil (10) nach einem der vorangehenden Ansprüche.

## Claims

1. Expiration valve (10) of a ventilator (100), comprising
a valve body (12) in which a fluid inlet (14) and a fluid outlet (16) for breathing air of a patient are formed, wherein the valve body (12) defines a fluid flow path between the fluid inlet (14) and the fluid outlet (16), the cross-section of which widens towards the fluid outlet (16);
wherein the valve body (12) forms a cup-shaped valve plenum (18) which is at least partially enclosed by a valve bottom (24) and into which the fluid inlet (14) opens; and
wherein the valve bottom (24) extends from an edge (26) of the fluid inlet (14) defining a fluid inlet surface (22);
**characterized in that**
the valve bottom (24) bulges in an axial direction in a region around the fluid inlet (14) with a bulge (32) having a depth varying around the circumference of the fluid inlet (14).

2. Expiration valve (10) according to claim 1, wherein the valve bottom (24) extends in a plate-like manner between the fluid inlet (14) and an outer edge of the valve body (12); wherein the valve bottom extends flat or flat conically between the fluid inlet and the outer edge.

3. Expiration valve (10) according to claim 1 or 2, wherein the valve bottom (24) has a rounded contour starting from an inner side of the fluid inlet (14) at the edge (26) of the fluid inlet surface (22) on its side facing the valve plenum (18).

4. Expiration valve (10) according to one of claims 1 to 3, wherein the depth of the bulge (32) increases in the circumferential direction towards the fluid outlet (16).

5. Expiration valve (10) according to one of claims 1 to 4, wherein the depth of the bulge (32) in the circumferential direction on a side opposite the fluid outlet (16) is smaller than in all other regions of the bulge.

6. Expiration valve (10) according to one of claims 1 to 5, wherein the depth of the bulge (32) in the circumferential direction is greater on a side facing the fluid outlet (16) than in all other regions of the bulge.

7. Expiration valve (10) according to one of claims 1 to 6, wherein the fluid inlet (14) is arranged in a central region of the valve plenum (18).

8. Expiration valve (10) according to claim 7, wherein the fluid outlet (16) is arranged further outside with respect to the fluid inlet (14).

9. Expiration valve (10) according to one of claims 1 to 8, wherein the valve body (12) is made of plastic material.

10. Expiration valve (10) according to one of claims 1 to 9, wherein the valve body (12) is an injection molded part.

11. Expiration valve (10) according to one of claims 1 to 10, wherein the valve body (12) comprises a fluid inlet part (28) in which the fluid inlet (14) is formed.

12. Expiration valve (10) according to claim 11, wherein the fluid inlet part (28) forms a first spigot-like projection; wherein the spigot-like projection is either cylindrically shaped or tapers towards a free end.

13. Expiration valve (10) according to one of claims 1 to 12, wherein the valve body (12) comprises at least one stiffening rib (36, 38) formed around the fluid inlet (14), in particular a plurality of stiffening ribs (36, 38) formed around the fluid inlet (14).

14. Expiration valve (10) according to claim 13, wherein the at least one stiffening rib (36, 38) is formed integrally with the valve body (12).

15. Expiration valve (10) according to claim 14, wherein the at least one stiffening rib (36, 38) extends outwardly away relative to the edge (26) of the fluid inlet (14).

16. Expiration valve (10) according to one of claims 13 to 15, wherein the at least one stiffening rib comprises at least a first stiffening rib (36) extending from the edge (26) of the fluid inlet (14) along the valve bottom (24).

17. Expiration valve (10) according to one of claims 13 to 16, wherein the at least one stiffening rib comprises at least one second stiffening rib (38) extending inwardly from the outer edge of the valve bottom (24) along the valve bottom (24).

18. Expiration valve (10) according to one of claims 1 to 17, wherein the valve body (12) forms a fluid outlet part (30) in which the fluid outlet (16) is formed.

19. Expiration valve (10) according to claim 18, wherein the bulging region (32) of the valve bottom (24) merges into the fluid outlet part (16).

20. Expiration valve according to one of the preceding claims, further comprising a valve membrane associated with the fluid inlet, the valve membrane being displaceable at least between a position closing the fluid inlet and a position opening the fluid inlet.

21. Expiration valve (10) according to one of claims 1 to 20, wherein the valve body (12) further comprises a support (42), on the upper side of which the valve membrane (20) can be placed with a contact surface formed on its outer edge, wherein the support (42) is formed unevenly on its upper side.

22. Ventilator (100), comprising an expiration valve (10) according to one of the preceding claims which can be connected to an expiration air duct (140).

## Revendications

1. Soupape d'expiration (10) d'un appareil respiratoire (100) présentant
un corps de soupape (12), dans lequel sont réalisées une entrée de fluide (14) et une sortie de fluide (16) pour l'air de respiration d'un patient, le corps de soupape (12) définissant une voie d'écoulement du fluide entre l'entrée de fluide (14) et la sortie de fluide (16), dont la section transversale s'élargit en direction de la sortie de fluide (16) ;
le corps de soupape (12) formant un plénum de soupape (18) en forme de pot, qui est au moins partiellement entouré par un fond de soupape (24) et dans lequel débouche l'entrée de fluide (14) ; et
le fond de soupape (24) partant d'un bord (26) de l'entrée de fluide (14) définissant la surface d'entrée de fluide (22) ;
**caractérisé en ce que**
le fond de soupape (24) se bombe dans une zone autour de l'entrée de fluide (14) dans la direction axiale avec un renflement (32) dont la profondeur varie autour de la circonférence de l'entrée de fluide (14).

2. La soupape d'expiration (10) selon la revendication 1, dans laquelle le fond de soupape (24) s'étend en forme d'assiette entre l'entrée de fluide (14) et un bord extérieur du corps de soupape (12) ; dans laquelle le fond de soupape s'étend à plat ou en cône plat entre l'entrée de fluide et le bord extérieur.

3. La soupape d'expiration (10) selon la revendication 1 ou 2, dans laquelle le fond de soupape (24) présente un contour arrondi sur son côté tourné vers le plénum de soupape (18) en partant d'un côté intérieur de l'entrée de fluide (14) au bord (26) de la surface d'entrée de fluide (22).

4. La soupape d'expiration (10) selon l'une quelconque des revendications 1 à 3, dans laquelle la profondeur du renflement (32) augmente dans la direction circonférentielle vers la sortie de fluide (16).

5. La soupape d'expiration (10) selon l'une quelconque des revendications 1 à 4, dans laquelle la profondeur du renflement (32), dans la direction circonférentielle, est plus petite sur un côté opposé à la sortie de fluide (16) que dans toutes les autres zones du renflement.

6. La soupape d'expiration (10) selon l'une quelconque des revendications 1 à 5, dans laquelle la profondeur du renflement (32), dans la direction circonférentielle, est plus grande sur un côté orienté vers la sortie de fluide (16) que dans toutes les autres zones du renflement.

7. La soupape d'expiration (10) selon l'une quelconque des revendications 1 à 6, dans laquelle l'entrée de fluide (14) est située dans une zone centrale du plénum de soupape (18).

8. La soupape d'expiration (10) selon la revendication 7, dans laquelle la sortie de fluide (16) est située plus à l'extérieur par rapport à l'entrée de fluide (14).

9. La soupape d'expiration (10) selon l'une quelconque des revendications 1 à 8, dans laquelle le corps de soupape (12) est réalisé en matière plastique.

10. La soupape d'expiration (10) selon l'une quelconque des revendications 1 à 9, dans laquelle le corps de soupape (12) est une pièce moulée par injection.

11. La soupape d'expiration (10) selon l'une quelconque des revendications 1 à 10, dans laquelle le corps de soupape (12) présente une pièce d'entrée de fluide (28) dans laquelle est réalisée l'entrée de fluide (14).

12. La soupape d'expiration (10) selon la revendication 11, dans laquelle la pièce d'entrée de fluide (28) forme un premier appendice en forme de tourillon ; dans laquelle l'appendice en forme de tourillon est soit de forme cylindrique, soit se rétrécit vers une extrémité libre.

13. La soupape d'expiration (10) selon l'une quelconque des revendications 1 à 12, dans laquelle le corps de soupape (12) présente au moins une nervure de renfort (36, 38) formée autour de l'entrée de fluide (14), notamment plusieurs nervures de renfort (36, 38) formées autour de l'entrée de fluide (14).

14. La soupape d'expiration (10) selon la revendication 13, dans laquelle la nervure de renfort (36, 38), une au moins, est formée d'un seul tenant avec le corps de soupape (12).

15. La soupape d'expiration (10) selon la revendication 14, dans laquelle la nervure de renfort (36, 38), une au moins, s'étend vers l'extérieur par rapport au bord (26) de l'entrée de fluide (14).

16. La soupape d'expiration (10) selon l'une quelconque des revendications 13 à 15, dans laquelle la nervure de renfort, une au moins, comprend au moins une première nervure de renfort (36), qui s'étend depuis le bord (26) de l'entrée de fluide (14) le long du fond de soupape (24).

17. La soupape d'expiration (10) selon l'une quelconque des revendications 13 à 16, dans laquelle la nervure de renfort, une au moins, comprend au moins une deuxième nervure de renfort (38), qui s'étend du bord extérieur du fond de soupape (24) vers l'intérieur, le long du fond de soupape (24).

18. La soupape d'expiration (10) selon l'une quelconque des revendications 1 à 17, dans laquelle le corps de soupape (12) forme une pièce de sortie de fluide (30) dans laquelle est réalisée la sortie de fluide (16).

19. La soupape d'expiration (10) selon la revendication 18, dans laquelle la zone de renflement (32) du fond de soupape (24) se confond avec la pièce de sortie de fluide (16).

20. La soupape d'expiration selon l'une quelconque des revendications précédentes, présentant en outre une membrane de soupape associée à l'entrée de fluide, qui peut être déplacée au moins entre une position fermant l'entrée de fluide et une position ouvrant l'entrée de fluide.

21. La soupape d'expiration (10) selon l'une des revendications 1 à 20, dans laquelle le corps de soupape (12) présente en outre un support (42) sur la face supérieure duquel la membrane de soupape (20) peut être posée avec une surface d'appui réalisée sur son bord extérieur, le support (42) étant réalisé de manière non plane sur sa face supérieure.

22. Appareil respiratoire (100) présentant une soupape d'expiration (10) pouvant être raccordée à une conduite d'air expiratoire (140) selon l'une quelconque des revendications précédentes.
